(19) Europäisches Patentamt
European Patent Office
Office européen des brevets

(11) Veröffentlichungsnummer: **0 223 140**
**A2**

(12) **EUROPÄISCHE PATENTANMELDUNG**

(21) Anmeldenummer: 86115191.8

(22) Anmeldetag: 03.11.86

(51) Int. Cl.⁴: C07D 498/04 , A23K 1/16 , C07D 333/38 , C07D 333/50 , //(C07D498/04,333:00,265:00)

(30) Priorität: 14.11.85 DE 3540377

(43) Veröffentlichungstag der Anmeldung: 27.05.87 Patentblatt 87/22

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Hallenbach, Werner, Dr.
Kleiststrasse 10
D-4018 Langenfeld(DE)
Erfinder: Lindel, Hans, Dr.
Carl-Duisberg-Strasse 321
D-5090 Leverkusen 1(DE)
Erfinder: Berschauer, Friedrich, Dr.
Claudiusweg 9
D-5600 Wuppertal 1(DE)
Erfinder: Scheer, Martin, Dr.
Herbert-Katernberg 7
D-5600 Wuppertal 1(DE)
Erfinder: De Jong, Anno, Dr.
Stockmannsmühle 46
D-5600 Wuppertal 1(DE)

(54) **Thienooxazinone, Verfahren zu ihrer Herstellung und ihre Verwendung als Leistungsförderer.**

(57) Die vorliegende Erfindung betrifft Thienooxazinone der Formel I

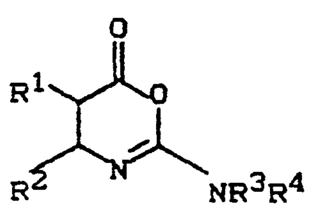

(I)

in welcher
$R^1$ und $R^2$ gemeinsam mit den angrenzenden C-Atomen für einen gegebenenfalls substituierten Thiophenring stehen,
$R^3$ für Wasserstoff, gegebenenfalls substituiertes Alkyl, Cycloalkyl, Alkenyl, Aryl steht,
$R^4$ für gegebenenfalls substituiertes Alkyl, Cycloalkyl, Alkenyl, Aryl steht,
$R^3$ und $R^4$ gemeinsam mit dem angrenzenden Stickstoffatom für einen gegebenenfalls substiuierten gesättigten Heterocyclus stehen, der gegebenenfalls weitere Heteroatome enthalten kann.

EP 0 223 140 A2

Xerox Copy Centre

Verfahren zu ihrer Herstellung und ihre Verwendung als Leistungsförderer bei Tieren.

## Thienooxazinone, Verfahren zu ihrer Herstellung und ihre Verwendung als Leistungsförderer

Die vorliegende Erfindung betrifft Thienooxazinone, Verfahren zu ihrer Herstellung und ihrer Verwendung als leistungsfördernde Mittel bei Tieren.

Benzoxazinone sind bekannt (vgl. DE-OS 2 315 303). Thienooxazinone stellen eine strukturell völlig neuartige Verbindungsklasse dar und können weder von ihrer Struktur noch von ihren Eigenschaften mit Benzoxazinonen verglichen werden.

Es wurde nun gefunden:

1. Neue Thienooxazinone der Formel I

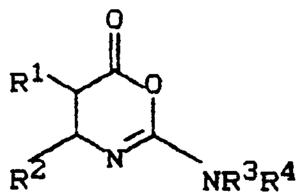

(I)

in welcher

$R^1$ und $R^2$ gemeinsam mit den angrenzenden C-Atomen für einen gegebenenfalls substituierten Thiophenring stehen,

$R^3$ für Wasserstoff, gegebenenfalls substituiertes Alkyl, Cycloalkyl, Alkenyl, Aryl steht,

$R^4$ für gegebenenfalls substituiertes Alkyl, Cycloalkyl, Alkenyl, Aryl steht,

$R^3$ und $R^4$ gemeinsam mit dem angrenzenden Stickstoffatom für einen gegebenenfalls substituierten gesättigten Heterocyclus stehen, der gebenenfalls weitere Heteroatome enthalten kann.

Die Thienooxazinone der Formel I können dabei in Form ihrer Isomeren der Formel Ia sowie als Gemische beider isomerer Formen vorliegen

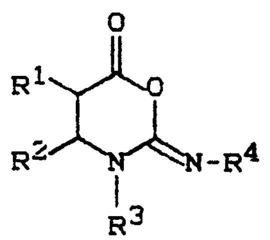

(Ia)

2. Verfahren zur Herstellung der neuen Thienooxazinone der allgemeinen Formel

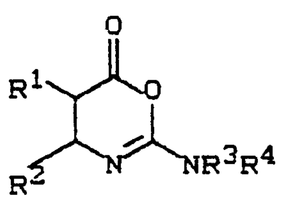

(I)

in welcher

$R^1$ und $R^2$ gemeinsam mit den angrenzenden C-Atomen für einen gegebenenfalls substituierten Thiophenring stehen,

$R^3$ für Wasserstoff, gegebenenfalls substituiertes Alkyl, Cycloalkyl, Alkenyl, Aryl steht,

$R^4$ für gegebenenfalls substituiertes Alkyl, Cycloalkyl, Alkenyl, Aryl steht,

$R^3$ und $R^4$ gemeinsam mit dem angrenzenden Stickstoffatom für einen gegebenenfalls substituierten

Heterocyclus stehen, der gegebenenfalls weitere Heteroatome enthalten kann, dadurch gekennzeichnet, daß man

a) Thienylharnstoffe der Formel II

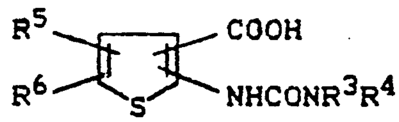

(II)

oder IIa

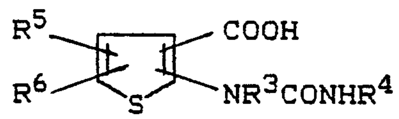

(IIa)

in welcher

$R^5$ und $R^6$ für gleiche oder verschiedene Reste aus der Gruppe Wasserstoff, Halogen, Nitro, CN, Alkyl, Alkoxy, Alkylthio, Aryl, Alkylcarbonyl, Alkoxycarbonyl, Arylcarbonyl, Aryloxycarbonyl, die gegebenenfalls substituiert sein können, stehen,

$R^5$ und $R^6$ können auch gemeinsam mit den angrenzenden C-Atomen für einen gegebenenfalls substituierten gesättigten oder ungesättigten carbocyclischen Ring stehen,

$R^3$ und $R^4$ die oben angegebene Bedeutung haben,

und die Reste -COOH, $-NHCONR^3R^4$ oder $-NR^3CONHR^4$ benachbart zueinander stehen,

mit Kondensationsmitteln umsetzt, oder

b) Aminothiophene der Formel III

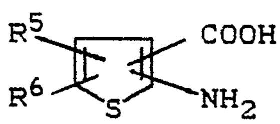

(III)

in welcher

$R^5$ und $R^6$ die oben angegebenen Bedeutung haben und

die Reste COOH und $NH_2$ benachbart zueinander stehen,.

mit mindestens 1 Mol (pro Mol Aminothiophen der Formel III) der Verbindungen der Formel IV

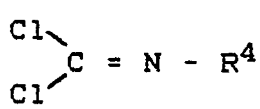

(IV)

in welcher

$R^4$ die oben angegebene Bedeutung hat umsetzt, oder

c) Verbindungen der Formel V

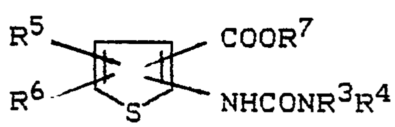

(V)

oder ihre Isomeren der Formel Va

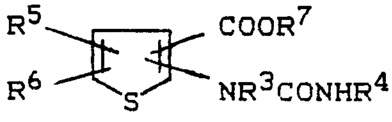

(Va)

in welcher
$R^3$, $R^4$, $R^5$, $R^6$ die oben angegebene Bedeutung besitzen und
$R^7$ für tertiäre Alkylreste, Benzyl steht,
mit sauren Kondensationsmitteln in Mischung mit wasserentziehenden Mitteln umsetzt.

3. Verbindungen der Formeln II und IIa

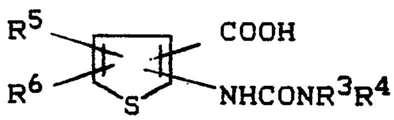

(II)

oder IIa

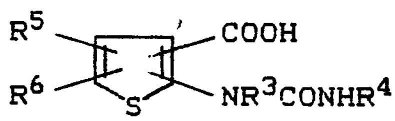

(IIa)

in welcher
$R^3$ für Wasserstoff, gegebenenfalls substituiertes Alkyl, Cycloalkyl, Alkenyl, Aryl steht,
$R^4$ für gegebenenfalls substituiertes Alkyl, Cycloalkyl, Alkenyl, Aryl steht,
$R^5$ und $R^6$ für gleiche oder verschiedene Reste aus der Gruppe Wasserstoff, Halogen, Nitro, CN, Alkyl, Alkoxy, Alkylthio, Aryl, Alkylcarbonyl, Alkoxycarbonyl, Arylcarbonyl, Aryloxycarbonyl, die gegebenenfalls substituiert sein können, stehen,
$R^5$ und $R^6$ können auch gemeinsam mit den angrenzenden C-Atomen für einen gegebenenfalls substituierten gesättigten oder ungesättigten carbocyclischen Ring, mit Ausnahme des unsubstituierten Phenylringes, stehen,
sind neu.

4. Verfahren zur Herstellung der Verbindungen der Formeln II und IIa gemäß Punkt 3 (oben), dadurch gekennzeichnet, daß man

a) Verbindungen der Formeln V und Va in welchen $R^3$, $R^4$, $R^5$, $R^6$, $R^7$ die unter Punkt 2c (oben) angegebenen Bedeutungen besitzen,
mit starken Säuren umsetzt oder

b) Verbindungen der Formel IV

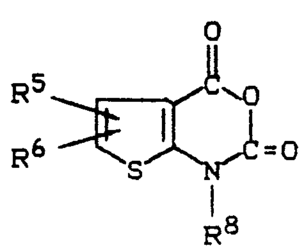

(VI)

in welcher
$R^5$ und $R^6$ die unter Punkt 3 angegebene Bedeutung besitzen,
$R^8$ für Wasserstoff oder gegebenenfalls substituiertes Alkyl, Cycloalkyl, Alkenyl, Aryl steht,

mit Aminen der Formel VII
H-$NR^3R^4$ (VII)
in welcher
$R^3$ und $R^4$ die unter Punkt 3 angegebene Bedeutung besitzen,
unter der Voraussetzung, daß für den Fall, daß
$R^8$ für gegebenenfalls substituiertes Alkyl, Cycloalkyl, Alkenyl, Aryl steht, der Rest
$R^3$ für Wasserstoff steht,
umsetzt.

Es war völlig überraschend, daß die Thienooxazinone der Formel I leistungsfördernde Eigenschaften bei Tieren aufweisen. Es gab aus dem Stand der Technik keinerlei Hinweis auf diese Verwendung dieser Verbindungsklasse.

Bevorzugt sind Thienooxazinone der Formel I, in welcher
$R^1$ und $R^2$ gemeinsam mit den angenzenden C-Atomen für einen Thiophenring stehen, der in 1,2-oder 2,3-Stellung an den Oxazinonring ankondensiert ist. Die Thiophenring ist durch die Reste $R^5$ und $R^6$ substituiert.
Bevorzugt steht
$R^5$ für Wasserstoff, Halogen, Nitro, CN, $C_{1-4}$-Alkoxy, $C_{1-4}$-Alkylthio, gegebenenfalls substituiertes $C_{1-6}$-Acyl, gegebenenfalls substituiertes Aroyl, insbesondere Benzoyl, für gegebenenfalls durch Halogen, $C_{1-4}$-Alkoxy, $C_{1-4}$-Alkylthio, Aryl, insbesondere Phe nyl, Aryloxy, insbesondere Phenoxy, Arylthio, insbesondere Phenylthio, Amino, $C_{1-4}$-Alkylamino, Di-$C_{1-4}$ -alkylamino, Arylamino, insbesondere Phenylamino substituiertes $C_{1-6}$-Alkyl, sowie für Phenyl steht, wobei die Phenylreste gegebenenfalls einen oder mehrere der folgenden Substituenten tragen: Halogen, $C_{1-4}$ -Alkyl, CN, $C_{1-4}$-Alkoxy, $C_{1-4}$-Alkylthio, Phenyl, Phenoxy, Phenylthio, Amino, $C_{1-4}$-Alkylamino, Di-$C_{1-4}$-alkylamino, $C_{1-4}$-Alkoxyalkyl, $C_{1-4}$-Halogenalkyl, $C_{1-4}$-Halogenalkoxy, $C_{1-4}$ -Halogenalkylthio, Methylendioxy oder Ethylendioxy, die gegebenenfalls halogensubstituiert sind, Acyl,
$R^6$ für die bei $R^5$ aufgeführten Reste,
$R^5$ und $R^6$ gemeinsam mit den angrenzenden beiden C-Atomen für gesättigte oder ungesättigte carbocyclische Reste mit 5-8 Ringgliedern, die gegebenenfalls durch OH, $C_{1-4}$-Alkyl, Halogen, Nitro, CN, $C_{1-4}$-Alkoxy, $C_{1-4}$-Alkylthio, Phenyl, Phenoxy, Phenylthio, Amino, $C_{1-4}$-Alkylamino, $C_{1-4}$-Dialkylamino, C $_{1-4}$-Halogenalkyl, $C_{1-4}$-Halogenalkoxy, $C_{1-4}$-Halogenalkylthio, $C_{1-4}$-Alkoxyalkyl substituiert sind,
$R^3$ für Wasserstoff,
$R^4$ für gegebenenfalls durch Halogen, $C_{1-4}$-Alkoxy, $C_{1-4}$-Alkylthio, Aryl, insbesondere Phenyl, Aryloxy, insbesondere Phenoxy, Arylthio, insbesondere Phenylthio, Amino, substituiertes $C_{1-4}$-Alkyl, ferner für Phenyl, wobei die Phenylreste gegebenenfalls einen oder mehrere der folgenden Substituenten tragen: Halogen, $C_{1-4}$-Alkyl, CN, $C_{1-4}$ -Alkoxy, $C_{1-4}$-Alkylthio, Phenyl, Phenoxy, Phenylthio, Amino, $C_{1-4}$-Alkylamino, Di-$C_{1-4}$-alkylamino, C $_{1-4}$-Halogenalkoxy, $C_{1-4}$-Halogenalkylthio, Methylendioxy oder Ethylendioxy, die gegebenenfalls halogensubstituiert sind, Acyl.

Besonders bevorzugt sind Verbindungen der Formel I, in welcher
$R^1$ und $R^2$ für einen in 1,2-Stellung an den Oxazinonring ankondensierten Thiophenring steht, der durch die Reste $R^5$ und $R^6$ substituiert ist.

Bevorzugt steht dabei
$R^5$ für Wasserstoff, $C_{1-6}$-Alkyl, das gegebenenfalls durch Fluor, Chlor oder Brom substituiert ist, Phenyl, das gegebenenfalls durch $C_{1-4}$-Alkyl, Halogen, $C_{1-4}$-Halogenalkyl, insbesondere Trifluormethyl, $C_{1-4}$-Halogenalkoxy, insbesondere Trifluormethoxy substituiert ist, für Nitro, Acyl, insbesondere Acetyl,
$R^6$ für die bei $R^5$ angegebenen Reste,
$R^5$ und $R^6$ gemeinsam mit den angrenzenden C-Atomen für einen gesättigten 5-8 gliedrigen carbocyclischen Ring stehen, der gegebenenfalls durch $C_{1-4}$-Alkyl substituiert ist, sowie gemeinsam mit den angrenzenden C-Atomen für einen annellierten Benzolring stehen, der gegbenenfalls durch Halogen, insbesondere Chlor, Nitro, $C_{1-4}$-Alkyl substituiert ist,
$R^3$ für Wasserstoff,
$R^4$ für $C_{1-6}$-Alkyl, Cycloalkyl mit bis zu 8 C-Atomen, Phenyl, das gegebenenfalls durch Halogen, insbesondere Chlor, Nitro, substituiert ist.

Insbesondere seien Verbindungen der Formel I genannt, in welcher
$R^1$ und $R^2$ gemeinsam mit den angrenzenden C-Atomen für einen Thiophenring stehen, der in 1,2-Stellung an den Oxazinonring ankondensiert ist und durch $R^5$ und $R^6$ substituiert ist. Bevorzugt steht
$R^5$ für Wasserstoff, $C_{1-4}$-Alkyl, insbesondere Methyl, Ethyl, t-Butyl, Acetyl, Phenyl, Nitro,
$R^6$ für die bei $R^5$ angeführten Reste,
$R^5$ und $R^6$ gemeinsam für einen an den Thiophenring ankondensierten Cyclopentan-, Cyclohexan-, Cycloheptan-, Cyclooctan-, Cyclohexanon-, Benzolring, die gegebenenfalls durch $C_{1-4}$ -Alkyl, insbesondere

Methyl, Halogen, insbesondere Chlor, Nitro substituiert sein können,
$R^3$ für Wasserstoff,
$R^4$ für $C_{1-6}$-Alkyl, insbesondere Methyl, Ethyl, Propyl, t-Butyl, Cycloalkyl mit bis zu 6 C-Atomen, Phenyl, das gegebenenfalls durch Halogen, insbesondere Chlor, substituiert ist.

Im einzelnen seien folgende Verbindungen der Formel I genannt:

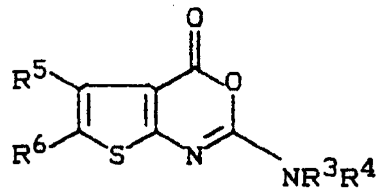

| $R^5$ | $R^6$ | $R^3$ | $R^4$ |
|---|---|---|---|
| H | 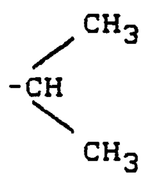 | H | $-CH_3$ |
| H | 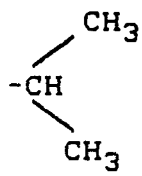 | H | 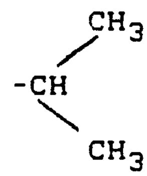 |
| H | 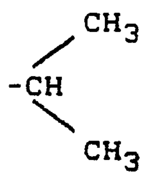 | H | 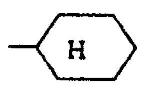 |
| H | 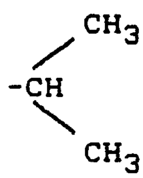 | H | 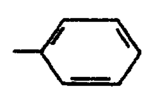 |
| H | 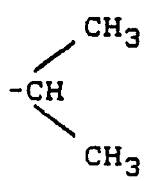 | H | sec-Butyl |
| H | 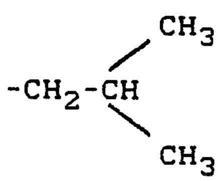 | H | $-CH_3$ |

| $R^5$ | $R^6$ | $R^3$ | $R^4$ |
|---|---|---|---|
| H | 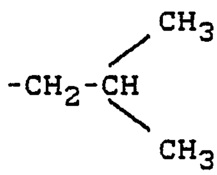 | H | 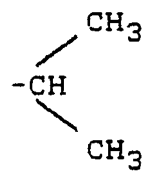 |
| H | 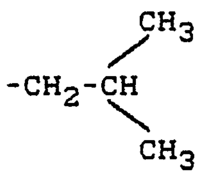 | H | 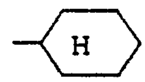 |
| H | 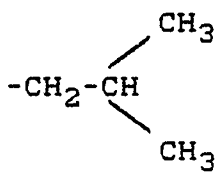 | H | 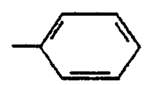 |
| H | 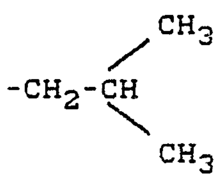 | H | sec-Butyl |
| H | 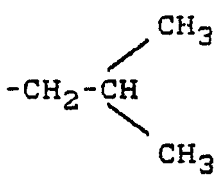 | H | tert.-Butyl |
| H | 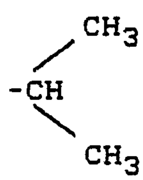 | H | tert.-Butyl |
| $-CH_3$ | -Et | H | 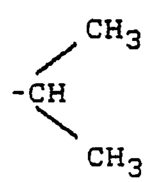 |

| $R^5$ | $R^6$ | $R^3$ | $R^4$ |
|---|---|---|---|
| $-CH_3$ | -Et | H | 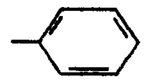 |
| $(CH_2)_3$ | | H | $CH_3$ |
| $(CH_2)_3$ | | H | i-Propyl |
| $(CH_2)_3$ | | H | n-Butyl |
| $(CH_2)_3$ | | H | Cyclohexyl |
| $(CH_2)_3$ | | H | Phenyl |
| $(CH_2)_3$ | | H | 4-Chlorphenyl |
| $(CH_2)_4$ | | H | $CH_3$ |
| $(CH_2)_4$ | | H | i-Propyl |
| $(CH_2)_4$ | | $CH_3$ | n-Butyl |
| $(CH_2)_4$ | | $CH_3$ | Cyclohexyl |
| $(CH_2)_4$ | | $C_2H_5$ | $CH_3$ |
| $(CH_2)_4$ | | $CH_3$ | Phenyl |
| $(CH_2)_4$ | | $CH_3$ | 4-Chlorphenyl |
| $(CH_2)_4$ | | $C_2H_5$ | i-Propyl |
| $(CH_2)_4$ | | $CH_3$ | 2-Methylphenyl |
| $(CH_2)_4$ | | $CH_3$ | 2-Methoxyphenyl |
| $(CH_2)_4$ | | $C_2H_5$ | n-Butyl |
| $(CH_2)_5$ | | $CH_3$ | $CH_3$ |
| $(CH_2)_5$ | | $CH_3$ | i-Propyl |
| $(CH_2)_5$ | | $C_2H_5$ | $CH_3$ |
| $(CH_2)_5$ | | $CH_3$ | n-Butyl |
| $(CH_2)_5$ | | $CH_3$ | Cyclohexyl |
| $(CH_2)_5$ | | $C_2H_5$ | i-Propyl |
| $(CH_2)_5$ | | $CH_3$ | Phenyl |
| $(CH_2)_5$ | | $C_2H_5$ | n-Butyl |

| $R^5$ | $R^6$ | $R^3$ | $R^4$ |
|---|---|---|---|
| H | $-CH_3$ | H | 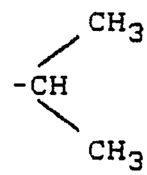 |
| H | $-CH_3$ | H | $-CH_3$ |
| H | $-CH_3$ | H | 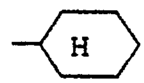 |
| H | $-CH_3$ | H | 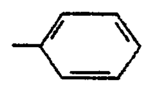 |
| 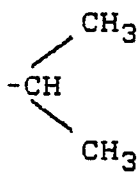 | H | H | $-CH_3$ |
| 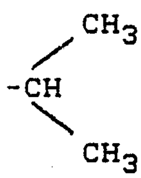 | H | H | 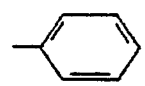 |
| 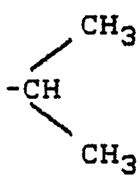 | H | H | 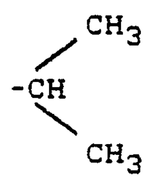 |
| H | -Et | H | $-CH_3$ |
| H | -Et | H | 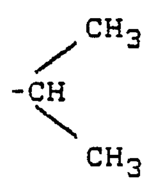 |

| $R^5$ | $R^6$ | $R^3$ | $R^4$ |
|---|---|---|---|
| H | -Et | H | 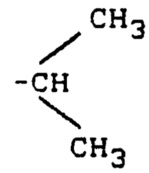 |
| H | -Et | H | 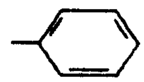 |
| H | -Et | H | tert.-Butyl |
| H | -Et | H | $-CH_3$ |
| -Et | $-CH_3$ | H | $-CH_3$ |
| $CH_3$ | H | H | $CH_3$ |
| $CH_3$ | H | H | i-Propyl |
| $CH_3$ | H | H | i-Butyl |
| $CH_3$ | H | H | Cyclopentyl |
| $CH_3$ | H | H | Cyclohexyl |
| $CH_3$ | H | H | Phenyl |
| $CH_3$ | H | H | 4-Methoxyphenyl |
| H | $n-C_5H_{11}$ | H | $CH_3$ |
| H | $n-C_5H_{11}$ | H | 1-Propyl |
| H | $n-C_5H_{11}$ | H | 1-Butyl |
| H | $n-C_5H_{11}$ | H | Cyclopentyl |
| H | $n-C_5H_{11}$ | H | Cyclohexyl |
| H | $n-C_5H_{11}$ | H | Phenyl |
| H | $n-C_5H_{11}$ | H | 4-Chlorphenyl |
| H | $n-C_5H_{11}$ | H | 4-Methoxyphenyl |
| H | Phenyl | H | Cyclopropyl |

Verwendet man 2-N'-Methylureido-3-carboxy-4,5-tetramethylen-thiopen als Ausgangsverbindung, läßt sich der Reaktionsverlauf bei Verfahren 2a zur Herstellung der Thienooxazinone wie folgt darstellen:

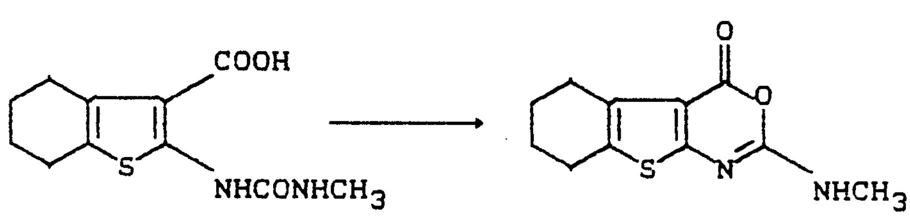

Als Ausgangsverbindungen der Formel II werden bevorzugt diejenigen eingesetzt, in denen die Reste $R^3$, $R^4$, $R^5$, $R^6$ die bei den Verbindungen der Formel I genannten bevorzugten Bedeutungen besitzen.

Die Verbindungen der Formel II sind neu. Ihre Herstellung wird weiter unten beschrieben.

Im einzelnen seien folgende Verbindungen der Formel II genannt:

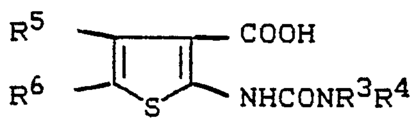

| $R^5$ | $R^6$ | $R^3$ | $R^4$ |
|---|---|---|---|
| H | 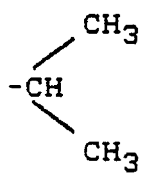 | H | $-CH_3$ |
| H | 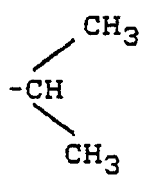 | H | 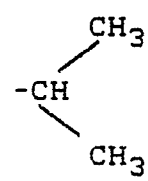 |
| H | 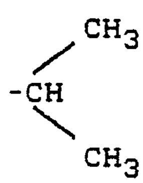 | H | 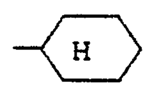 |
| H | 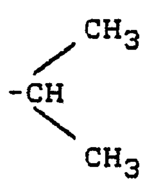 | H | 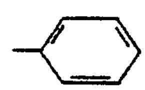 |
| H | 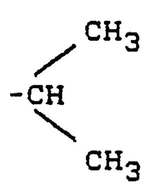 | H | sec-Butyl |
| H | 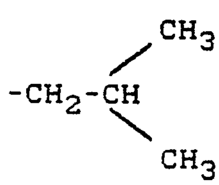 | H | $-CH_3$ |

| $R^5$ | $R^6$ | $R^3$ | $R^4$ |
|---|---|---|---|
| H | 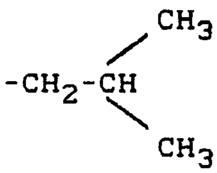 | H | 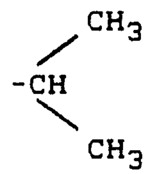 |
| H | 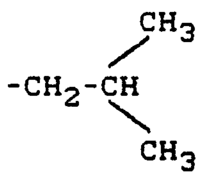 | H | 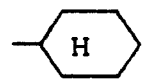 |
| H | 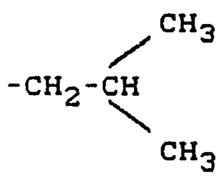 | H | 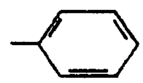 |
| H | 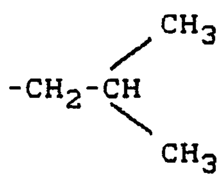 | H | sec-Butyl |
| H | 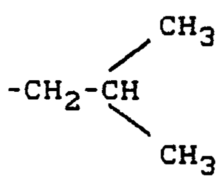 | H | tert.-Butyl |
| H | 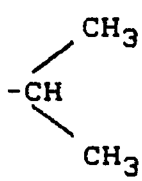 | H | tert.-Butyl |
| $-CH_3$ | -Et | H | 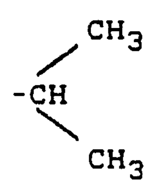 |

| $R^5$ | $R^6$ | $R^3$ | $R^4$ |
|---|---|---|---|
| $-CH_3$ | -Et | H | 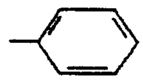 |
| $(CH_2)_3$ | | H | $CH_3$ |
| $(CH_2)_3$ | | H | i-Propyl |
| $(CH_2)_3$ | | H | n-Butyl |
| $(CH_2)_3$ | | H | Cyclohexyl |
| $(CH_2)_3$ | | H | Phenyl |
| $(CH_2)_3$ | | H | 4-Chlorphenyl |
| $(CH_2)_4$ | | H | $CH_3$ |
| $(CH_2)_4$ | | H | i-Propyl |
| $(CH_2)_4$ | | $CH_3$ | n-Butyl |
| $(CH_2)_4$ | | $CH_3$ | Cyclohexyl |
| $(CH_2)_4$ | | $C_2H_5$ | $CH_3$ |
| $(CH_2)_4$ | | $CH_3$ | Phenyl |
| $(CH_2)_4$ | | $CH_3$ | 4-Chlorphenyl |
| $(CH_2)_4$ | | $C_2H_5$ | i-Propyl |
| $(CH_2)_4$ | | $CH_3$ | 2-Methylphenyl |
| $(CH_2)_4$ | | $CH_3$ | 2-Methoxyphenyl |
| $(CH_2)_4$ | | $C_2H_5$ | n-Butyl |
| $(CH_2)_5$ | | $CH_3$ | $CH_3$ |
| $(CH_2)_5$ | | $CH_3$ | i-Propyl |
| $(CH_2)_5$ | | $C_2H_5$ | $CH_3$ |
| $(CH_2)_5$ | | $CH_3$ | n-Butyl |
| $(CH_2)_5$ | | $CH_3$ | Cyclohexyl |
| $(CH_2)_5$ | | $C_2H_5$ | i-Propyl |
| $(CH_2)_5$ | | $CH_3$ | Phenyl |
| $(CH_2)_5$ | | $C_2H_5$ | n-Butyl |

| $R^5$ | $R^6$ | $R^3$ | $R^4$ |
|---|---|---|---|
| H | $-CH_3$ | H | 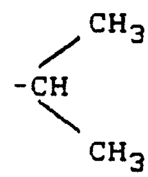 |
| H | $-CH_3$ | H | $-CH_3$ |
| H | $-CH_3$ | H | 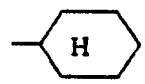 |
| H | $-CH_3$ | H | 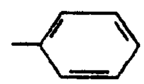 |
| 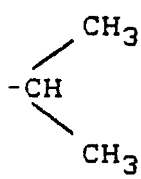 | H | H | $-CH_3$ |
| 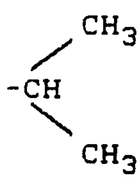 | H | H | 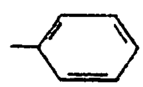 |
| 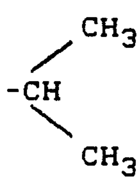 | H | H | 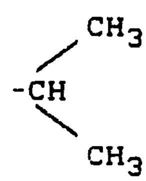 |
| H | -Et | H | $-CH_3$ |
| H | -Et | H | 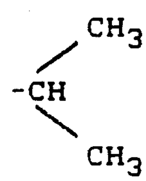 |

| $R^5$ | $R^6$ | $R^3$ | $R^4$ |
|---|---|---|---|
| H | -Et | H | 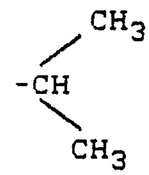 |
| H | -Et | H | 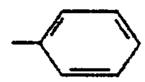 |
| H | -Et | H | tert.-Butyl |
| H | -Et | H | $-CH_3$ |
| -Et | $-CH_3$ | H | $-CH_3$ |
| $CH_3$ | H | H | $CH_3$ |
| $CH_3$ | H | H | i-Propyl |
| $CH_3$ | H | H | i-Butyl |
| $CH_3$ | H | H | Cyclopentyl |
| $CH_3$ | H | H | Cyclohexyl |
| $CH_3$ | H | H | Phenyl |
| $CH_3$ | H | H | 4-Methoxyphenyl |
| H | $n-C_5H_{11}$ | H | $CH_3$ |
| H | $n-C_5H_{11}$ | H | 1-Propyl |
| H | $n-C_5H_{11}$ | H | 1-Butyl |
| H | $n-C_5H_{11}$ | H | Cyclopentyl |
| H | $n-C_5H_{11}$ | H | Cyclohexyl |
| H | $n-C_5H_{11}$ | H | Phenyl |
| H | $n-C_5H_{11}$ | H | 4-Chlorphenyl |
| H | $n-C_5H_{11}$ | H | 4-Methoxyphenyl |
| H | Phenyl | H | Cyclopropyl |

Als Kondensationsmittel für die Umsetzung sind geeignet niedere aliphatische Carbonsäureanhydride z.B. Acetanhydrid, Propionsäureanhydrid, Buttersäureanhydrid; halogensubstituierte aliphatische Carbonsäureanhydride wie Trifluoressigsäureanhydrid; sowie ferner Dicyclohexylcarbodiimid.

Die Umsetzung kann im überschüssigen Kondensationsmittel selbst oder in Verdünnung mit einem geeigneten Verdünnungsmittel durchgeführt werden.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl-und Dibutylether, Glykoldimethylether und Diglykoldimethylether, Tetrahydrofuran und Dioxan, weiterhin Ketone, wie

Aceton, Methylethyl-, Methylisopropyl-und Methylisobutylketon, außerdem Ester, wie Essigsäuremethylester und -ethylester, ferner Nitrile, wie z.B. Acetonitril und Propionitril, Benzonitril, Glutarsäuredinitril Tetramethylensulfon und Hexamethylphosphorsäuretriamid. Darüber hinaus niedere aliphatische Carbonsäuren wie Essigsäure, Propionsäure, Buttersäure, Trifluoressigsäure.

Katalysatoren für die Cyclisierung sind nicht unbedingt erforderlich. In vielen Fällen wird jedoch durch Zugabe einer starken Säure ein Beschleunigung der Reaktion erreicht.

Geeignete Säuren sind z.B. HCl, $H_2SO_4$, Trifluoressigsäure, Toluolsulfonsäure.

Die Cyclisierung kann in einem Temperaturbereich von -20 bis +150°C durchgeführt werden, bevorzugt im Temperaturbereich von 0 -100°C.

Normalerweise arbeitet man unter Normaldruck, jedoch kann es zweckmäßig sein, z.B. beim Einsatz niedrig siedender Lösungsmittel unter Druck zu arbeiten.

Bei der Durchführung des erfindungsgemäßen Verfahrens setzt man mindestens 1 Mol des Kondensationsmittels pro Mol Ureidothiophencarbonsäure ein. Bevorzugt beim Arbeiten in Verdünnungsmitteln ist ein molares Verhältnis von 1-3:1, insbesondere 1-1,5:1. Ist das Kondensationsmittel zugleich Reaktionsmedium, so sind 3 -30 Mol, insbesondere 3 -5 Mol Kondensationsmittel pro Mol Ureidothiophencarbonsäure anzuwenden.

Die Reaktionsprodukte werden isoliert, indem man aus den entsprechenden Lösungsmitteln direkt ausfallende Produkte filtriert oder indem man das Lösungsmittel abdestilliert.

Verwendet man bei Verfahren 2b) 3-Amino-4-carboxy-5-methylthiophen und Phenylisocyaniddichlorid als Ausgangsverbindungen, läßt sich der Reaktionsverlauf wie folgt darstellen:

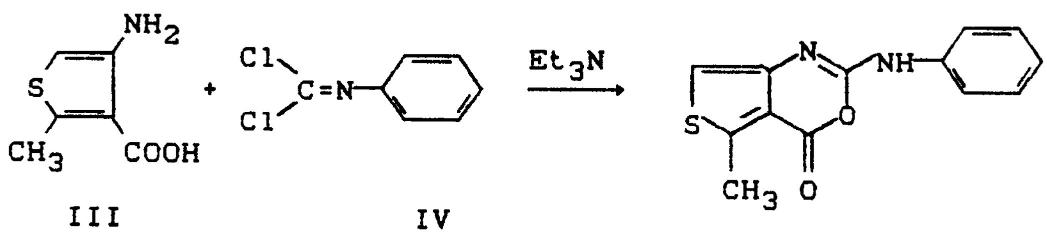


III IV

Die Aminothiophene der Formel III sind bekannt oder lassen sich analog zu bekannten Verfahren darstellen (K. Gewald et al. Chem. Ber. 98 (1965) S. 3571; Chem. Ber. 99 (1966) S. 94; EP-OS 4 931).

Bevorzugt sind Aminothiophene der Formel III, in welcher die Reste $R^5$ und $R^6$ die bei den Verbindungen der Formel I genannten bevorzugten Bedeutungen besitzen.

Im einzelnen seien genannt:

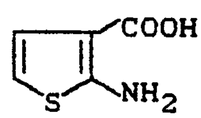

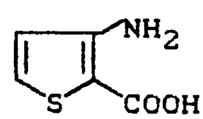

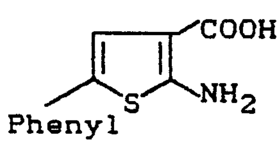

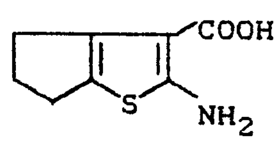

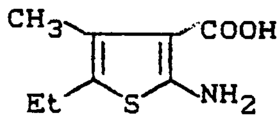

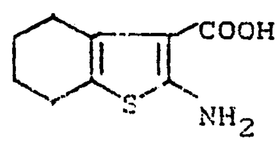

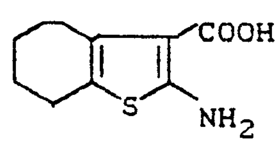

Verbindungen der Formel IV sind bekannt oder lassen sich analog zu bekannten Verfahren herstellen. Bevorzugt sind Verbindungen der Formel IV, in welcher R' die bei den Verbindungen der Formel I genannten bevorzugten Bedeutungen besitzt.

Im einzelnen seien genannt:
Methylisocyaniddichlorid, Ethylisocyaniddichlorid, Propylisocyaniddichlorid, Isopropylisocyaniddichlorid, n-Butylisocyaniddichlorid, Isobutylisocyaniddichlorid, tert.-Butylisocyaniddichlorid, Cyclohexylisocyaniddichlorid, Phenylisocyaniddichlorid.

Die erfindungsgemäße Umsetzung zwischen den Aminothiophenen der Formel III und den Verbindungen der Formel IV führt man vorzugsweise in Gegenwart eines Verdünnungsmittels durch. Als solche eignen sich alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl-und Dibutylether, Glykoldimethylether und Diglykoldimethylether, Tetrahydrofuran und Dioxan, weiterhin Ketone, wie Aceton, Methylethyl-, Methylisopropyl-und Methylisobutylketon, außerdem Ester, wie Essigsäuremethylester und -ethylester, ferner Nitrile, wie z.B. Acetonitril und Propionitril, Benzonitril, Glutarsäuredinitril, Tetramethylensulfon und Hexamethylphosphorsäuretriamid.

Die Umsetzung erfolgt in Gegenwart von Säureakzeptoren.

Als Säureakzeptoren können alle üblichen Säurebindemittel verwendet werden. Hierzu gehören vorzugsweise Alkalicarbonate, -hydroxide oder -alkoholate, wie Natrium-oder Ka liumcarbonat, Natrium-und Kaliumhydroxid, Natrium-und Kaliummethylat bzw. -ethylat, ferner aliphatische, aromatische oder heterocyclische Amine, beispielsweise Trimethylamin, Triethylamin, Tributylamin, Dimethylanilin, Dimethylbenzylamin, Pyridin und 4-Dimethylaminopyridin.

Zur Beschleunigung des Reaktionsverlaufs können Katalysatoren zugesetzt werden. Als solche können Verbindungen verwendet werden, welche gewöhnlich bei Reaktionen in Zweiphasensystemen aus Wasser und mit Wasser nicht mischbaren organischen Lösungsmitteln zum Phasentransfer von Reaktanden dienen (Phasentransferkatalysatoren). Als solche sind vor allem Tetraalkyl-und Trialkylaralkyl-ammoniumsalze mit vorzugsweise 1 bis 10, insbesondere 1 bis 8 Kohlenstoffen je Alkylgruppe, vorzugsweise Phenyl als Arylbestandteil der Aralkylgruppe und vorzugsweise 1 bis 4, insbesondere 1 oder 2 Kohlenstoffatomen im Alkylteil der Aralkylgruppen bevorzugt. Hierbei kommen vor allem die Halogenide, wie Chloride, Bromide und Iodide, vorzugsweise die Chloride und Bromide in Frage. Beispielhaft seien Tetrabutylammoniumbromid, Benzyl-triethylammoniumchlorid und Methyltrioctylammoniumchlorid genannt. Außerdem 4-Dimethylaminopyridin sowie 4-Pyrrolidino-pyridin.

Die Reaktionstemperaturen können in einem größeren Temperaturbereich variiert werden. Im allgemeinen arbeitet man zwischen 0°C und 120°C, vorzugsweise zwischen 20°C und 70°C.

Normalerweise arbeitet man unter Normaldruck, jedoch kann es zweckmäßig sein, z.B. beim Einsatz niedrig siedender Isocyaniddichloride, in geschlossenen Gefäßen unter Druck zu arbeiten.

Bei der Durchführung des erfindungsgemäßen Verfahrens setzt man mindestens 1 Mol der Verbindung der Formel IV pro Mol Aminothiophen ein. Bevorzugt ist ein molares Verhältnis von Verbindungen der Formel IV zu Aminothiophen wie 1-3:1, insbesondere 1-1,5:1. Die Säureakzeptoren werden mindestens in Mengen von 2 Mol Säureakzeptoren pro Mol Isocyaniddichlorid zugesetzt. Bevorzugt ist ein molares Verhältnis von Säureakzeptoren zu Isocyaniddichlorid wie 2-6:1, insbesondere 2-3:1.

Die Katalysatoren werden vorzugsweise in Mengen von 0,01 bis 0,1 Mol pro Mol Aminothiophen angewandt.

Die Reaktionsprodukte werden isoliert, indem man aus den entsprechenden Lösungsmitteln direkt ausfallende Produkte filtriert oder indem man das Lösungsmittel abdestilliert.

Verwendet man bei Verfahren 2c 2-N'-Isopropylureido-3-tertiär-butoxycarbonyl-4,5-dimethyl-thiophen und ein Gemisch aus Trifluoressigsäure und Trifluoressigsäureanhydrid, so läßt sich der Reaktionsverlauf wie folgt darstellen:

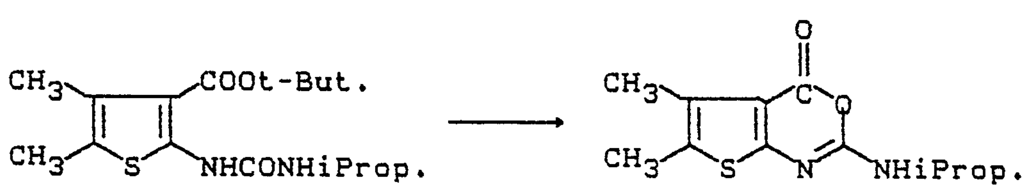

Als Ausgangsverbindungen der Formel V und Va werden bevorzugt diejenigen eingesetzt, in denen die Reste $R^3$, $R^4$, $R^5$, $R^6$ die bei den Verbindungen der Formel I genannten bevorzugten Bedeutungen besitzen.

$R^7$ steht bevorzugt für t-Butyl oder Benzyl.

Im einzelnen seien folgende Verbindungen der Formel V genannt:

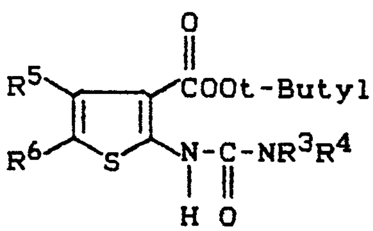

| $R^5$ | $R^6$ | $R^3$ | $R^4$ |
|---|---|---|---|
| H | 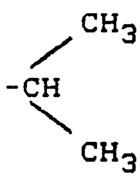 | H | $-CH_3$ |
| H | 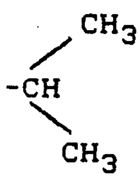 | H | 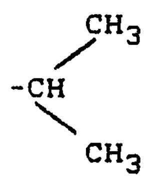 |
| H | 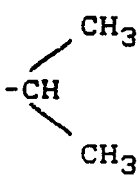 | H | 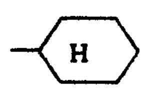 |
| H | 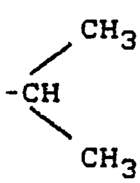 | H | 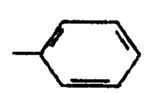 |

| $R^5$ | $R^6$ | $R^3$ | $R^4$ |
|---|---|---|---|
| H | 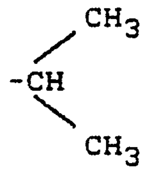 | H | sec-Butyl |
| H | 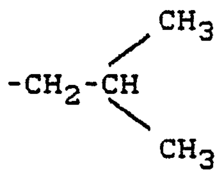 | H | $-CH_3$ |
| H | 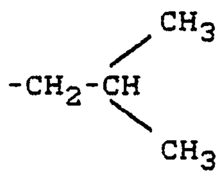 | H | 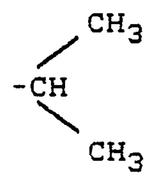 |
| H | 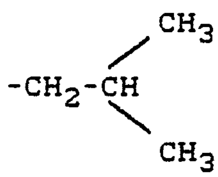 | H | 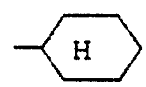 |
| H | 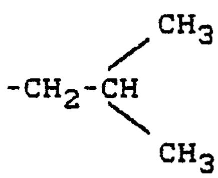 | H | 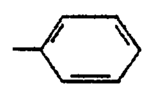 |
| H | 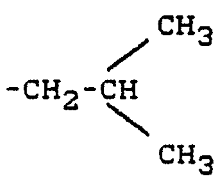 | H | sec-Butyl |
| H | 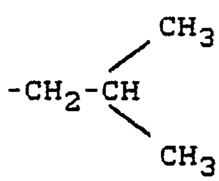 | H | tert.-Butyl |

| $R^5$ | $R^6$ | $R^3$ | $R^4$ |
|---|---|---|---|
| H | 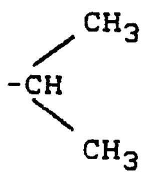 | H | tert.-Butyl |
| $-CH_3$ | -Et | H | 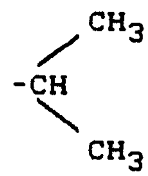 |
| $-CH_3$ | -Et | H | 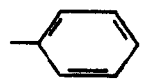 |
| $(CH_2)_3$ | | H | $CH_3$ |
| $(CH_2)_3$ | | H | i-Propyl |
| $(CH_2)_3$ | | H | n-Butyl |
| $(CH_2)_3$ | | H | Cyclohexyl |
| $(CH_2)_3$ | | H | Phenyl |
| $(CH_2)_3$ | | H | 4-Chlorphenyl |
| $(CH_2)_4$ | | H | $CH_3$ |
| $(CH_2)_4$ | | H | i-Propyl |
| $(CH_2)_4$ | | $CH_3$ | n-Butyl |
| $(CH_2)_4$ | | $CH_3$ | Cyclohexyl |
| $(CH_2)_4$ | | $C_2H_5$ | $CH_3$ |
| $(CH_2)_4$ | | $CH_3$ | Phenyl |
| $(CH_2)_4$ | | $CH_3$ | 4-Chlorphenyl |
| $(CH_2)_4$ | | $C_2H_5$ | i-Propyl |
| $(CH_2)_4$ | | $CH_3$ | 2-Methylphenyl |
| $(CH_2)_4$ | | $CH_3$ | 2-Methoxyphenyl |
| $(CH_2)_4$ | | $C_2H_5$ | n-Butyl |
| $(CH_2)_5$ | | $CH_3$ | $CH_3$ |

| $R^5$ | $R^6$ | $R^3$ | $R^4$ |
|---|---|---|---|
| $(CH_2)_5$ | | $CH_3$ | i-Propyl |
| $(CH_2)_5$ | | $C_2H_5$ | $CH_3$ |
| $(CH_2)_5$ | | $CH_3$ | n-Butyl |
| $(CH_2)_5$ | | $CH_3$ | Cyclohexyl |
| $(CH_2)_5$ | | $C_2H_5$ | i-Propyl |
| $(CH_2)_5$ | | $CH_3$ | Phenyl |
| $(CH_2)_5$ | | $C_2H_5$ | n-Butyl |
| $(CH_2)_4$ | | H | n-Butyl |
| $(CH_2)_4$ | | H | Cyclohexyl |
| $(CH_2)_4$ | | H | Phenyl |
| $(CH_2)_4$ | | H | 4-Methoxyphenyl |
| $(CH_2)_5$ | | H | $CH_3$ |
| $(CH_2)_5$ | | H | i-Propyl |
| $(CH_2)_5$ | | H | Cyclohexyl |
| $(CH_2)_5$ | | H | Phenyl |
| $(CH_2)_5$ | | H | 4-Chlorphenyl |
| H | $-CH_3$ | H | 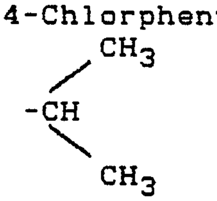 |
| H | $-CH_3$ | H | $-CH_3$ |
| H | $-CH_3$ | H | 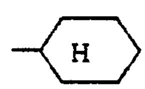 |
| H | $-CH_3$ | H | 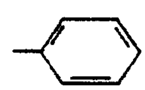 |
| 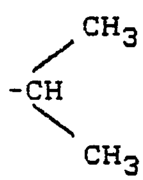 | H | H | $-CH_3$ |

| $R^5$ | $R^6$ | $R^{73}$ | $R^4$ |
|---|---|---|---|
| 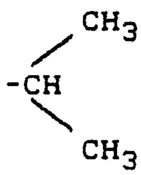 | H | H | 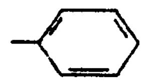 |
| 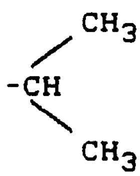 | H | H | 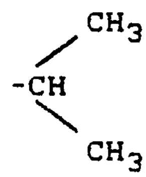 |
| H | -Et | H | $-CH_3$ |
| H | -Et | H | 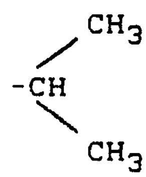 |
| H | -Et | H | 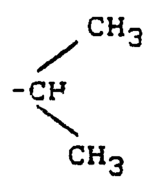 |
| H | -Et | H | 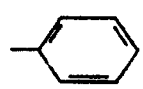 |
| H | -Et | H | tert.-Butyl |
| H | -Et | H | $-CH_3$ |
| -Et | $-CH_3$ | H | $-CH_3$ |

| $R^5$ | $R^6$ | $R^{73}$ | $R^4$ |
|---|---|---|---|
| $CH_3$ | H | H | $CH_3$ |
| $CH_3$ | H | H | i-Propyl |
| $CH_3$ | H | H | i-Butyl |
| $CH_3$ | H | H | Cyclopentyl |
| $CH_3$ | H | H | Cyclohexyl |
| $CH_3$ | H | H | Phenyl |
| $CH_3$ | H | H | 4-Methoxyphenyl |
| H | $n-C_5H_{11}$ | H | $CH_3$ |
| H | $n-C_5H_{11}$ | H | 1-Propyl |
| H | $n-C_5H_{11}$ | H | 1-Butyl |
| H | $n-C_5H_{11}$ | H | Cyclopentyl |
| H | $n-C_5H_{11}$ | H | Cyclohexyl |
| H | $n-C_5H_{11}$ | H | Phenyl |
| H | $n-C_5H_{11}$ | H | 4-Chlorphenyl |
| H | $n-C_5H_{11}$ | H | 4-Methoxyphenyl |
| H | Phenyl | H | Cyclopropyl |

Die Verbindungen der Formel V und Va sind z.T. bekannt und z.T. Gegenstand einer älteren nicht vorveröffentlichten deutschen Patentanmeldung der Anmelderin, DE-P 3 517 706.3. Sie werden z.B. erhalten, indem man in an sich bekannter Weise entsprechend substituierte Thienylamine mit Isocyanaten oder mit Phosgen und Aminen umsetzt oder indem man entsprechend substituierte Thienylisocyanate mit Aminen umsetzt. Die Herstellung der Verbindungen der Formel V und Va kann auch analog zu den in DE-OS 2 122 636 und 2 627 935 beschriebenen Verfahren erfolgen.

Die Umsetzung erfolgt in Gegenwart saurer Kondensationsmittel in Kombination mit einem wasserentziehenden Mittel. Saure Kondensationsmittel sind z.B. konzentrierte anorganische Säuren wie z.B. Salzsäure, Schwefelsäure, Phosphorsäure, Polyphosphorsäure, starke organische Säuren wie z.B. Trifluoressigsäure.

Wasserentziehende Mittel sind z.B. Trifluoracetanhydrid, Acetanhydrid, Carbodiimide wie z.B. Dicyclohexylcarbodiimid.

Die Umsetzung erfolgt in Gegenwart von Verdünnungsmitteln. Als solche eignen sich alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Dietheyl-und Dibutylether, Glykoldimethylether und Diglykoldimethylether, Tetrahydrofuran und Dioxan, weiterhin Ketone, wie Aceton, Methylethyl-, Methylisopropyl-und Methylisobutylketon, außerdem Ester, wie Essigsäuremethylester und -ethylester, ferner Nitrile, wie z.B. Acetonitril und Propionitril, Benzonitril, Glutarsäuredinitril, darüber hinaus Tetramethylensulfon und Hexamethylphosphorsäuretriamid.

Die Umsetzung erfolgt bei Temperaturen von -70°C bis +100°C, bevorzugt bei -10°C bis +60°C.

Die Umsetzung erfolgt bei Normaldruck.

Im allgemeinen setzt man pro Mol Verbindung der Formel V oder Va 1 Mol Kondensationsmittel ein. Bevorzugt sind Trifluoracetanhydrid, Acetanhydrid.

Bevorzugt wird die Reaktion in einem Gemisch aus Trifluoressigsäure und Trifluoressigsäureanhydrid im Verhältnis 1:1 durchgeführt.

Die Aufarbeitung nach beendeter Reaktion erfolgt durch Eingießen in Wasser und Abfiltrieren, evtl. nach vorheriger Neutralisation.

Verwendet man bei Verfahren 4a 5-Brom-3-tert.-butoxycarbonyl-2-(N'-isobutyl-ureido)-thiophen und Trifluoressigsäure als Ausgangssubstanzen, läßt sich der Reaktionsverlauf (zur Herstellung der Verbindungen der Formeln II oder IIa) wie folgt darstellen:

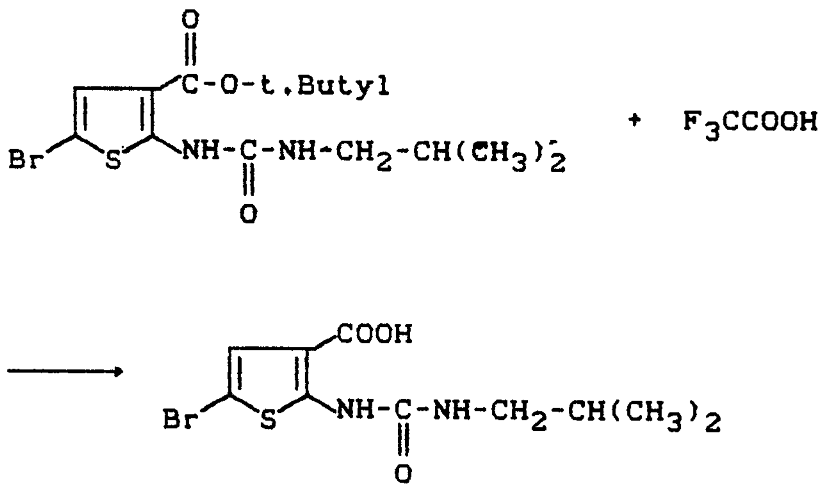

Die tert.-Butoxycarbonyl-ureidothiophene sind bekannt oder lassen sich analog zu bekannten Verfahren darstellen (vgl. Le A 23 725, 24 004).

Bevorzugt werden als Ausgangsverbindungen Ureidothiophene der Formeln V oder Va eingesetzt, in welchen die Reste $R^3$, $R^4$, $R^5$, $R^6$ die bei den Verbindungen der Formel 1 genannten bevorzugten Bedeutungen besitzen und der Rest $R^7$ für tert.-Butyl oder Benzyl steht.

Im einzelnen seien die weiter oben genannten Verbindungen der Formel V genannt.

Die Abspaltung der Esterreste gelingt vor allem mit starken Säuren wie z.B. Salzsäure, Bromwasserstoff, Schwefelsäure, Toluolsulfonsäure, Methansulfonsäure, Trifluoressigsäure und Mischungen derselben mit niederen aliphatischen Carbonsäuren wie z.B. Ameisensäure, Essigsäure, Propionsäure.

Die Umsetzung kann in der verwendeten Säure als Reaktionsmittel oder in Gegenwart von Verdünnungsmitteln durchgeführt werden.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl-und Dibutylether, Glykoldimethylether und Diglykoldimethylether, Tetrahydrofuran und Dioxan, weiterhin Ketone, wie Aceton, Methylethyl-, Methylisopropyl-und Methylisobutylketon, außerdem Ester, wie Essigsäuremethylester und -ethylester.

Die Reaktionstemperatur wird zwischen etwa 0°C und 130°C, vorzugsweise zwischen etwa 20°C und 60°C gehalten. Das Verfahren wird vorzugsweise bei Normaldruck durchgeführt, jedoch kann es zweckmäßig sein, z.B. bei Verwendung flüchtiger Säuren, unter Druck zu arbeiten.

Die Produkte werden isoliert, indem man aus den entsprechenden Lösungsmitteln ausgefallene Produkte abfiltriert oder das Lösungsmittel abdestilliert.

Verwendet man bei Verfahren 4b 5-Methyl-thieno[2,3-d]-oxazin-dion und Isobutylamin, so läßt sich die Reaktion durch folgendes Schema darstellen:

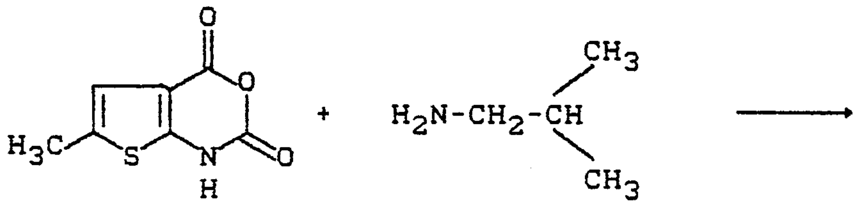

VI VII

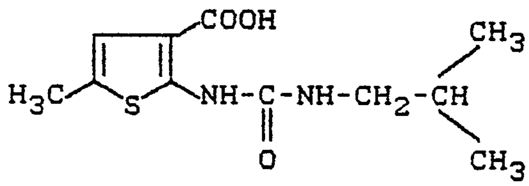

Thienooxazindione der Formel VI sind bekannt oder lassen sich nach bekannten Verfahren darstellen - (vgl. G.M.Coppola et al, J. Heterocycl. Chem. 19, 717 (1982), BE 852 328).

Bevorzugt sind Thienooxazindione, in welchen die Reste $R^5$ und $R^6$ die bei den Verbindungen der Formel I genannten bevorzugten Bedeutungen besitzen und $R^8$ für Wasserstoff steht.

Im einzelnen seien folgende Verbindungen der Formel IV genannt:

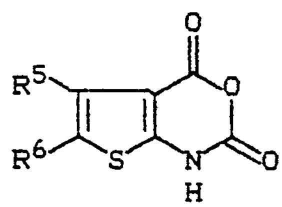

| $R^5$ | $R^6$ |
|---|---|
| H | $-CH(CH_3)_2$ |
| H | $-CH_3$ |
| H | $-CH_2-CH(CH_3)_2$ |
| $-CH_3$ | $-CH_3$ |
| $-CH_3$ | $-C_2H_5$ |
| $-CH_3$ | H |
| H | 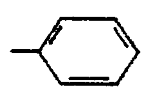 |
| H | $-C_2H_5$ |
| $-(CH_2)_3-$ | |
| $-(CH_2)_4-$ | |
| $-(CH_2)_5-$ | |

Verbindungen der Formel VII sind bekannt oder lassen sich nach bekannten Verfahren herstellen. Bevorzugt sind Verbindungen der Formel VII, in welchen $R^3$ und $R^4$ die bei den Verbindungen der Formel I genannten bevorzugten Bedeutungen besitzen.

Beispielhaft seien gennant:
Methylamin, Dimethylamin, Ethylamin, Diethylamin, n-Propylamin, Di-n-propylamin, Isopropylamin, Diisopropylamin, n-Butylamin, i-Butylamin, sec.-Butylamin, t.-Butlyamin, Cyclopentylamin, Cyclohexylamin, Anilin, 2-Chloranilin, 3-Chloranilin, 4-Chloranalin, 2-Nitroanalin, 3-Nitroanilin, 4-Nitroanilin, 2-Methylanilin, 3-Methylanilin, 4-Methylanilin, 2-Methoxyanilin, 3-Methoxyanilin, 4-Methoxyanilin, 2-Trifluormethylanilin, 3-Trifluormethylanilin, 4-Trifluormethylanilin.

Zur Herstellung der Thienylharnstoffe der Formel II und IIa werden die Thienooxazindione der Formel VI mit mindestens äquimolaren Mengen des Amins der Formel VII umgesetzt. Die Umsetzung kann mit oder ohne Verdünnungsmittel erfolgen. Beim Arbeiten ohne Verdünnungsmittel wird das Amin bevorzugt mit 3 - 30 Mol pro Mol Thienooxazindion, besonders bevorzugt 3 -10 Mol eingesetzt. Wird in Gegenwart von Verdünnungsmittel gearbeitet, werden bevorzugt 1 -3 Mol, besonders bevorzugt 1 -2 Mol Amin pro Mol Thienooxazindion angewendet.

Als Verdünnungsmittel seien genannt:
Alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl-und Dibutylether, Glykoldimethylether und Diglykoldimethylether, Tetrahydrofuran und Dioxan, weiterhin Ketone, wie Aceton, Methylethyl-, Methylisopropyl-und Methylisobutylketon, außerdem Ester, wie Essigsäuremethylester und -ethylester, ferner Nitrile, wie z.B. Acetonitril und Propionitril, Benzonitril, Glutarsäuredinitril, darüber hinaus Amide, wie. z.B. Dimethylformamid, Dimethylacetamid und N-Methylpyrrolidon, sowie Dimethylsulfoxid, Tetramethylensulfon und Hexamethylphosphorsäuretriamid.

Zur Beschleunigung des Reaktionsverlaufs können Katlaysatoren zugesetzt werden. Als solche sind geeignet: z.B. tertiäre Amine wie Pyridin, 4-Dimethylaminopyridin, Triethylamin, Triethylendiamin, Trimethylen-tetrahydropyrimidin; ferner Zinn-II-und Zinn-IV-Verbindngen wie Zinn-II-octoat oder Zinn-IV-chlorid -Die als Reaktionsbeschleuniger genannten tertiären Amine, z.B. Pyridin, können auch als Lösungsmittel verwendet werden.

Die Reaktionstemperaturen können in einem größeren Temperaturbereich variiert werden. Im allgemeinen arbeitet man zwischen 0°C und 120°C, vorzugsweise zwischen 20°C und 70°C.

Normalerweise arbeitet man unter Normaldruck, jedoch kann es zweckmäßig sein, z.B. beim Einsatz niederig siedender Amine, in geschlossenen Gefäßen unter Druck zu arbeiten.

Die Katalysatoren werden vorzugsweise in Mengen von 0,01 bis 0,1 Mol pro Mol der Reaktionskomponenten angewandt, jedoch sind auch größere Mengen, z.B. der tertiären Amine, anwendbar.

Die Reaktionsprodukte werden isoliert, indem man aus den entsprechenden Lösungsmitteln direkt ausfallende Produkte filtriert oder indem man das Lösungsmittel abdestilliert.

Die Wirkstoffe werden als Leistungsförderer bei Tieren zur Förderung und Beschleunigung des Wachstums, der Milch-und Wollproduktion, sowie zur Verbesserung der Futterverwertung, der Fleischqualität und zur Verschiebung des Fleisch-Fett-Verhältnisses zugunsten von Fleisch eingesetzt. Die Wirkstoffe werden bei Nutz-, Zucht-, Zier-und Hobbytieren verwendet.

Zu den Nutz-und Zuchttieren zählen Säugetiere wie z.B. Rinder, Schweine, Pferde, Schafe, Ziegen, Kaninchen, Hasen, Damwild, Pelztiere wird Nerze, Chinchilla, Geflügel wie z.B. Hühner, Puten, Gänse, Enten, Tauben, Fische wie z.B. Karpfen, Forellen, Lachse, Aale, Schleien, Hechte, Reptilien wie z.B. Schlangen und Krokodile.

Zu den Zier-und Hobbytieren zählen Säugetiere wie Hunde unt Katzen, Vögel wie Papageien, Kanarienvögel, Fische wie Zier-und Aquarienfische z.B. Goldfische.

Die Wirkstoffe werden unabhängig vom Geschlecht der Tiere während allen Wachstums-und Leistungsphasen der Tiere engesetzt. Bevorzugt werden die Wirkstoffe während der intensiven Wachstums-und Leistungsphase eingesetzt. Die intensive Wachstums-und Leistungsphase dauert je nach Tierart von einem Monat bis zu 10 Jahren.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise, bei etwa 0,001 bis 50 mg/kg insbesondere 0,01 bis 5 mg/kg Körpergewicht pro Tag. Die passende Menge des Wirkstoffs sowie die passende Dauer der Verabreichung hängen insbesondere von der Art, dem Alter, dem Geschlecht, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verarbreichung hängt insbesondere von der Art, dem Verhalten und dem Gesundheitszustand der Tiere ab.

Die Wirkstoff können einmalig verabreicht werden. Die Wirkstoffe können aber auch während der ganzen oder während eines Teils der Wachstumsphase temporär oder kontinuierlich verabreicht werden. Bei kontinuierlicher Verabreichung kann die Anwendung ein-oder mehrmals täglich in regelmäßigen oder unregelmäsigen Abständen erfolgen.

Die Verabreichung erfolgt oral oder parenteral in dafür geeigneten Formulierungen oder in reiner Form. Orale Formulierungen sind Pulver, Tabletten, Granulate, Doenche, Bolie sowie Futtermittel, Prämixe für Futtermittel, Formulierungen zur Verabreichung über Trinkwasser.

Die oralen Formulierungen enthalten den Wirkstoff in Konzentrationen von 0.01 ppm -100 %, bevorzugt von 0,01 ppm -1%.

Parenterale Formulierungen sind Injektionen in Form von Lösungen, Emulsionen und Suspensionen, sowie Implantate.

Die Wirkstoffe können in den Formulierungen allein oder in Mischung mit anderen Wirkstoffen, Mineralsalzen, Spurenelementen, Vitaminen, Eiweißstoffen, Farbstoffen, Fetten oder Geschmacksstoffen vorliegen.

Die Konzentration der Wirkstoffe im Futter beträgt normalerweise etwa 0,01-500 ppm, bevorzugt 0,1-50 ppm.

Die Wirkstoffe können als solche oder in Form von Prämixen oder Futterkonzentraten dem Futter zugesetzt werden.

Beispiel für die Zusammensetzung eines Kükenaufzuchtfutters, das erfindungsgemäßen Wirkstoff enthält:

200 g Weizen, 340 g Mais, 361 g Sojaschrot, 60 g Rindertalg, 15 g Dicalciumphosphat, 10 g Calciumcarbonat, 4 g jodiertes Kochsalz, 7.5 g Vitamin-Mineral-Mischung und 2,5 g Wirkstoff-Prämix ergeben nach sorgfältigem Mischen 1 kg Futter.

In einem kg Futtermischung sind enthalten:

600 I.E. Vitamin A, 100 I.E. Vitamin, $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg Mn $SO_2$ x $H_2O$, 140 mg Zn $SO_4$ x 7 $H_2O$, 100 mg Fe $SO_4$ x 7 $H_2O$ und 20 mg Cu $SO_4$ x 5 $H_2O$.

2,5 g Wirkstoff-Prämix enthalten z.B. 10 mg Wirkstoff, 1 g DL-Methionin, Rest Sojabohnenmehl.

Beispiel für die Zusammensetzung eines Schweineaufzuchtfutters, das erfindungsgemäßen Wirkstoff enthält:

630 g Futtergetreideschrot (zusammengesetzt aus 200 g Mais, 150 g Gerste-, 150 g Hafer-und 130 Weizenschrot), 80 g Fischmehl, 60 g Sojaschrot, 60 g Tapiokamehl, 38 g Bierhefe, 50 g Vitamin-Mineral-Mischung für Schweine, 30 g Leinkuchenmehl, 30 g Maiskleberfutter, 10 g Sojaöl, 10 g Zuckerrohrmelasse und 2 g Wirkstoff-Prämix (Zusammensetzung z.B. wie beim Kükenfutter) ergeben nach sorgfältigem Mischen 1 kg Futter.

Die angegebenen Futtergemische sind zur Aufzucht und Mast von vorzugsweise Küken bzw. Schweinen abgestimmt, sie können jedoch in gleicher oder ähnlicher Zusammensetzung auch zur Fütterung anderer Tiere verwendet werden.

Beispiel A

Ratten-Fütterungsversuch

Weibliche Laborratten 90-110 g schwer vom Typ SPF Wistar (Züchtung Hagemann) werden ad lib mit Standard Rattenfutter, das mit der gewünschten Menge Wirkstoff versetzt ist, gefüttert. Jeder Versuchsansatz wird mit Futter der identischen Charge durchgeführt, so daß Unterschiede in der Zusammensetzung des Futters die Vergleichbarkeit der Ergebnisse nich beeinträchtigen können.

Die Ratten erhalten Wasser ad lib.

Jeweils 12 Ratten bilden eine Versuchsgruppe und werden mit Futter, das mit der gewünschten Menge Wirkstoff versetzt ist gefüttert. Eine Kontrollgruppe erhält Futter ohne Wirkstoff. Das durchschnittliche Körpergewicht sowie die Streuung in den Körpergewichten der Ratten ist in jeder Versuchsgruppe gleich, so daß eine Vergleichbarkeit der Versuchsgruppen untereinander gewährleistet ist.

Während des 13-tägigen Versuchs werden Gewichtszunahme und Futterverbrauch bestimmt.

Es werden die aus der Tabelle ersichtlichen Ergebnisse erhalten:

<u>Tabelle</u>: Ratten-Fütterungsversuch

| Wirkstoff | Dosis 25 ppm | Gewichtszunahme |
|---|---|---|
| Kontrolle, ohne Wirkstoff | | 100 |
| 16 | | 104 |
| 19 | | 104 |
| 21 | | 102 |
| 4 | | 119 |
| 5 | | 113 |

Herstellung der Verbindungen der Formel 1 gemäß Verfahren 2c:

Beispiel 1(c)

In eine Mischung von 7,34 g (35 mmol) Trifluoracetanhydrid und 32 ml Trifluoressigsäure wurden 11 g -(32 mmol) 4,5-Dimethyl-3-tert.-butoxycarbonyl-2-N'-phenylureidothiophen unter Rühren bei Raumtemperatur eingetragen und anschließend noch eine Stunde nachgerührt. Zur Aufarbeitung wurde unter gutem Rühren in 500 ml gesättigte $NaHCO_3$-Lösung eingetropft und der Niederschlag abgesaugt.

Zur Reinigung wird aus Toluol umkristallisiert.

Ausbeute: 5,2 g (60 % Theorie)

Schmelzpunkt 227°C (wird sofort wieder fest).

Analog werden die folgenden Beispiele erhalten:

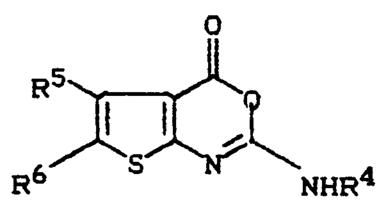

| Bsp.-Nr. | $R^5$ | $R^6$ | $R^4$ | Fp |
|---|---|---|---|---|
| 2 | $CH_3$ | $CH_3$ | $CH_3$ | 198° C (Zers.) |
| 3 | $CH_3$ | $CH_3$ | $i\text{-}C_3H_7$ | 200° C (Zers.) |
| 4 | $CH_3$ | $CH_3$ | $i\text{-}C_4H_9$ | 172 |

| Bsp.-Nr. | $R^5$ | $R^6$ | $R^4$ | Fp(° C) |
|---|---|---|---|---|
| 5 | H | H | $-CH_3$ | 196 (Zers.) |
| 6 | H | H | i-Propyl | 172 |
| 7 | H | H | i-Butyl | 162 |
| 8 | H | H | 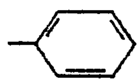 | 185 |
| 9 | H | H | t-Butyl | 168 |
| 10 | H | $CH_3$ | $-CH_3$ | 210 (Zers.) |
| 11 | H | $CH_3$ | i-Propyl | 190 |
| 12(c) | H | $CH_3$ | i-Butyl | 172 |

| Bsp.-Nr. | $R^4$ | $R^5$-$R^6$ | Fp(° C) |
|---|---|---|---|
| 13 | $CH_3$ | $(CH_2)_4$ | 195 |
| 14 | $i-C_3H_7$ | $(CH_2)_3$ | 206 |
| 15 | $t-C_4H_9$ | $(CH_2)_3$ | 167 |
| 16 | $2-CH_3O-C_6H_4$ | $(CH_2)_3$ | 206 |
| 17 | $t-C_4H_9$ | $(CH_2)_4$ | 209 |
| 18 | $2-CH_3O-C_6H_4$ | $(CH_2)_4$ | 182-3 |
| 19 | $i-C_3H_7$ | $(CH_2)_5$ | 226 |
| 20 | $t-C_4H_9$ | $(CH_2)_5$ | 191 |
| 21 | $i-C_3H_7$ | $(CH_2)_4$ | 203 |
| 22 | $n-C_4H_9$ | $(CH_2)_3$ | 185 |

Herstellung der Verbindungen der Formel I gemäß Verfahren 2b:

Beispiel 1(b)

1,15 g (6,7 mmol) 4,5-Dimethyl-2-aminothiophen-3-carbonsäure wurden in 20 ml trockenem Chloroform gelöst und 1,7 g (16,9 mmol) Triethylamin sowie 1,27 g (7,3 mmol) Phenylisocyaniddichlorid zugegeben. Anschließend wurde 8 Stunden unter Rückfluß gekocht. Zur Aufarbeitung wurden 100 ml Wasser zugegeben, die organische Phase abgetrennt und noch dreimal mit je 100 ml 5 %iger $NaH_2PO_4$-Lösung gewa-

schen. Das Lösungsmittel wurde im Vakuum abdestilliert und der Rückstand an Kieselgel mit Dichlormethan als Laufmittel chromatographiert.
Ausbeute: 190 mg (10,4 % der Theorie)
Schemlzpunkt: 226-7° (wird sofort wieder fest).

Herstellung der Verbindungen der Formel I gemäß Verfahren 2a:

Beispiel 12(a)

Eine Suspension von 588 mg (2,3 mmol) 3-Carboxy-5-methyl-2-(N'-isobutylureido)-thiophen und 0,26 g (2,5 mmol) Acetanhyrid in 5 ml trockenem Toluol wurde 8 Stunden unter Rühren auf 60°C erwärmt. Zur Aufarbeitung wurden 50 ml $CHCl_3$ zugegeben, zweimal mit $NaHCO_3$-Lösung gewaschen und bis zur beginnenden Kristallisation eingedampft. Es wurden 50 ml Petrolether zugegeben, 15 Minuten stehen gelassen und abgesaugt,
Ausbeute: 300 mg (54,8 % der Theorie)
Schmelzpunkt: 173°.

Herstellung der Ausgangsprodukte

Beispiel a)

Herstellung der Verbindungen der Formel II gemäß Verfahren 4a
800 mg (2,56 mmol) 3-t-Butoxycarbonyl-2-(N'-isobutylureido)-5-methyl-thiopen wurden zu einer Mischung aus 2 ml Trifluoressigsäure und 5 ml Dichlormethan gegeben und über Nacht gerührt. Danach wurde in Vakuum eingedampft, zuletzt an der Ölpumpe.
Ausbeute: 643 mg (98 % der Theorie)
Schmelzpunkt: 188-9°C (Zersetzung).

Beispiel b)

Herstellung der Verbindungen der Formel II gemäß Verfahren 4b
5 g (27,3 mmol) 5-Methylthieno[2,3-d]-3-H-oxazin-2,7-dion und 3,5 g (59 mmol) Isopropylamin werden in 30 ml trockenem DMF gelöst und 2 Stunden bei Raumtemperatur gerührt. Zur Aufarbeitung wird auf 200 ml Wasser gegossen, mit verdünnter HCl angesäuert und mit $CHCl_3$ extrahiert. Das Lösungsmittel wird im Vakuum abdestilliert und der Rückstand aus Toluol umkristallisiert.
Ausbeute: 1.39 g (21 % der Theorie)
Schmelzpunkt: 181°C (Zersetzung)

Herstellung der Verbindungen der Formel V

Beispiel c1)

4,5-Dimethyl-3-tert.-butoxycarbonyl-2-N'-isobutylureido-thiophen

Zu 16 g (70,5 mmol) 4,5-Dimethyl-3-tert.-butoxy-carbonyl-2-aminothiophen in 200 ml trockenem Pyridin wurden 14,3 g (0,144 mol) Isobutylisocyanat zugegeben und 18 Stunden bei 50°C gerührt. Nach Abkühlung wurde in 1 l 2,5 N wäßrige Salzsäure eingerührt und der fest anfallende Harnstoff abgesaugt.

Die Reinigung erfolgte durch Umkristallisieren aus Ethanol.
Ausbeute: 6,9 g (30 % der Theorie)
Schmelzpunkt: 173°C

Beispiel c2)

4,5-Dimethyl-3-tert.-butoxycarbonyl-2-N'-phenylureido-thiophen

Zu einer Lösung von 10 g (44 mmol) 4,5-Dimethyl-3-tert.-butoxycarbonyl-2-aminothiophen und 9,8 g (97 mmol) Triethylamin in 200 ml trockenem Chloroform wurden bei -10°C 24 ml (46 mmol) einer 1,93 M Phosgenlösung in Toluol zugetropft. Nach beendeter Zugabe wurde noch 15 Minuten ohne Kühlung gerührt, dann 4,5 g (48 mmol) Anilin zugetropft und noch eine Stunde bei Raumtemperatur gerührt. Zur Aufarbeitung wurde auf 1 l Wasser gegossen, die organische Phase abgetrennt und zweimal mit je 200 ml 5 %iger wäßriger $NaH_2PO_4$-Lösung gewaschen. Nach Trocknung mit $Na_2SO_4$ wurde das Lösungsmittel im Vakuum abdestilliert und der Rückstand aus Toluol umkristallisiert.

Ausbeute: 11,8 g (77 % der Theorie)

Schmelzpunkt: 218°C

Analog einem dieser Verfahren wurden die folgenden Verbindungen erhalten:

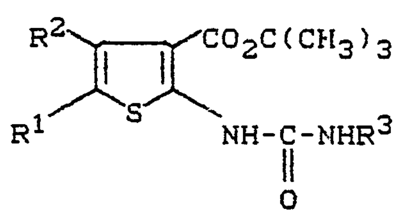

| Bsp.-Nr. | $R^1$ | $R^2$ | $R^3$ | Fp. |
|---|---|---|---|---|
| c3 | $CH_3$ | $CH_3$ | $CH_3$ | 149 |
| c4 | $CH_3$ | $CH_3$ | $i\text{-}C_3H_7$ | 186 |
| c5 | H | H | $CH_3$ | 160 |
| c6 | H | H | $i\text{-}C_3H_7$ | 207 |

| Bsp.-Nr. | $R^1$ | $R^2$ | $R^3$ | Fp. |
|---|---|---|---|---|
| c7 | $(CH_2)_3$ | | $i\text{-}C_3H_7$ | 182 |
| c8 | $(CH_2)_4$ | | $CH_3$ | 150 |
| c9 | $(CH_2)_4$ | | $i\text{-}C_3H_7$ | 182 |
| c10 | $(CH_2)_5$ | | $i\text{-}C_3H_7$ | 193 |

## Ansprüche

1. Thienooxazinone der Formel I

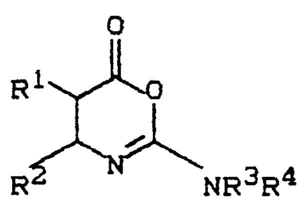

(I)

in welcher
$R^1$ und $R^2$ gemeinsam mit den angrenzenden C-Atomen für einen gegebenenfalls substituierten Thiophenring stehen,
$R^3$ für Wasserstoff, gegebenenfalls substituiertes Alkyl, Cycloalkyl, Alkenyl, Aryl steht,
$R^4$ für gegebenenfalls substituiertes Alkyl, Cycloalkyl, Alkenyl, Aryl steht,
$R^3$ und $R^4$ gemeinsam mit dem angrenzenden Stickstoffatom einen gesättigten Heterocyclus, der gegebenenfalls weitere Heteroatome enthalten kann, bilden
Die Thienooxazinone der Formel I können dabei in Form ihrer Isomeren der Formel Ia sowie als Gemische beider isomerer Formen vorliegen

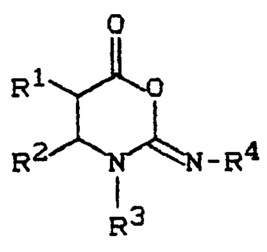

(Ia)

2. Verfahren zur Herstellung der neuen Thienooxazinone der allgemeinen Formel

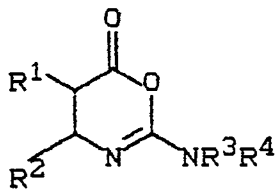

(I)

in welcher
$R^1$ und $R^2$ gemeinsam mit den angrenzenden C-Atomen für einen gegebenenfalls substituierten Thiophenring stehen,
$R^3$ für Wasserstoff, gegebenenfalls substituiertes Alkyl, Cycloalkyl, Alkenyl, Aryl steht,
$R^4$ für gegebenenfalls substituiertes Alkyl, Cycloalkyl, Alkenyl, Aryl steht,
$R^3$ und $R^4$ gemeinsam mit dem angrenzenden Stickstoffatom für einen gegebenenfalls substituierten Heterocyclus stehen, der gegebenenfalls weitere Heteroatome enthalten kann,
dadurch gekennzeichnet, daß man
a) Thienylharnstoffe der Formel II

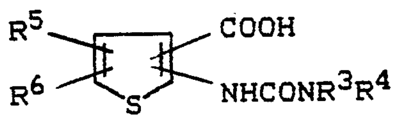

(II)

oder IIa

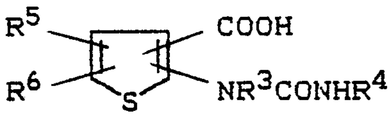

(IIa)

in welcher
$R^5$ und $R^6$ für gleiche oder verschiedene Reste aus der Gruppe Wasserstoff, Halogen, Nitro, CN, Alkyl, Alkoxy, Alkylthio, Aryl, Alkylcarbonyl, Alkoxycarbonyl, Arylcarbonyl, Aryloxycarbonyl, die gegebenenfalls substituiert sein können, stehen,
$R^5$ und $R^6$ können auch gemeinsam mit den angrenzenden C-Atomen für einen gegebenenfalls substituierten gesättigten oder ungesättigten carbocyclischen Ring stehen,
$R^3$ und $R^4$ die oben angegebene Bedeutung haben,
und die Reste -COOH, $-NHCONR^3R^4$ oder $-NR^3CONHR^4$ benachbart zueinander stehen,
mit Kondensationsmitteln umsetzt, oder
b) Aminothiophene der Formel III

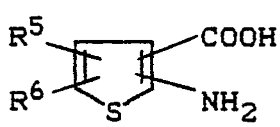

(III)

in welcher
$R^5$ und $R^6$ die oben angegebene Bedeutung haben und
die Reste COOH und $NH_2$ benachbart zueinander stehen,
mit mindestens 1 Mol (pro Mol Aminothiophen der Formel III) der Verbindungen der Formel IV

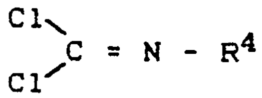

(IV)

in welcher
$R^4$ die oben angegebene Bedeutung hat, umsetzt, oder

c) Verbindungen der Formel V

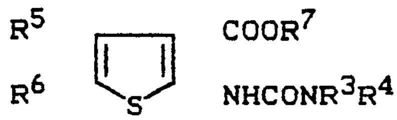

(V)

oder ihre Isomeren der Formel Va

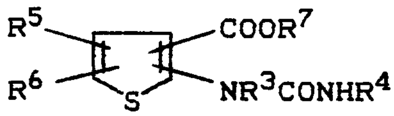

(Va)

in welcher
$R^3$, $R^4$, $R^5$, $R^6$ die oben angegebene Bedeutung besitzen und
$R^7$ für tertiäre Alkylreste, Benzyl steht,
mit sauren Kondensationsmitteln in Mischung mit wasserentziehenden Mitteln umsetzt.

3.Verbindungen der Formeln II und IIa

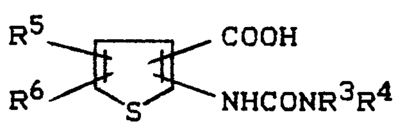

(II)

oder IIa

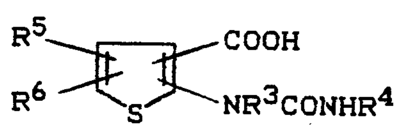

(IIa)

in welcher
$R^3$ für Wasserstoff, gegebenenfalls substituiertes Alkyl, Cycloalkyl, Alkenyl, Aryl steht,
$R^4$ für gegebenenfalls substituiertes Alkyl, Cycloalkyl, Alkenyl, Aryl steht,
$R^5$ und $R^6$ für gleiche oder verschiedene Reste aus der Gruppe Wasserstoff, Halogen, Nitro, CN, Alkyl, Alkoxy, Alkylthio, Aryl, Alkylcarbonyl, Alkoxycarbonyl, Arylcarbonyl, Aryloxycarbonyl, die gegebenenfalls substituiert sein können, stehen,
$R^5$ und $R^6$ können auch gemeinsam mit den angrenzenden C-Atomen für einen gegebenenfalls substituierten gesättigten oder ungesättigten carbocyclischen Ring, mit Ausnahme des unsubstituierten Phenylringes, stehen.

4. Verfahren zur Herstellung der Verbindungen der Formeln II und IIa gemäß Punkt 3 (oben), dadurch gekennzeichnet, daß man

a) Verbindungen der Formeln V und Va in welchen $R^3$, $R^4$, $R^5$, $R^6$, $R^7$ die unter Punkt 2c (oben) angegebenen Bedeutungen besitzen,
mit starken Säuren umsetzt oder

b) Verbindungen der Formel IV

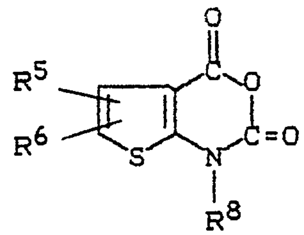

(VI)

in welcher

$R^5$ und $R^6$ die unter Punkt 3 angegebene Bedeutung besitzen,

$R^8$ für Wasserstoff oder gegebenenfalls substituiertes Alkyl, Cycloalkyl, Alkenyl, Aryl steht,

mit Aminen der Formel VII

$H\text{-}NR^3R^4$ (VII)

in welcher

$R^3$ und $R^4$ die unter Punkt 3 angegebene Bedeutung besitzen,

unter der Voraussetzung, daß für den Fall, daß

$R^8$ für gegebenenfalls substituiertes Alkyl, Cycloalkyl, Alkenyl, Aryl steht, der Rest

$R^3$ für Wasserstoff steht,

umsetzt.

5. Mittel zur Leistungsförderung von Tieren gekennzeichnet durch einen Gehalt an Tienooxazinonen der Formel I gemäß Anspruch 1.

6. Tierfutter, Trinkwasser für Tiere, Zusätze für Tierfutter und Trinkwasser für Tiere gekennzeichnet durch einen Gehalt an Thienooxazinon der Formel I gemäß Anspruch 1.

7. Verwendung von Thienoxazinonen der Formel I gemäß Anspruch 1 zur Leistungsförderung von Tieren.

8. Verfahren zur Herstellung von Mitteln zur Leistungsförderung von Tieren, dadurch gekennzeichnet, daß man Thienooxazinone der Formel I gemäß Anspruch 1 mit Streck-und/oder Verdünnungsmitteln vermischt.

9. Verfahren zur Herstellung von Tierfutter, Trinkwasser für Tiere oder Zusätze für Tierfutter und Trinkwasser für Tiere, dadurch gekennzeichnet, daß man Thienoxazinone der Formel I gemäß Anspruch 1 mit Futtermitteln oder Trinkwasser und gegebenenfalls weiteren Hilfsstoffen vermischt.